# EUROPEAN PATENT SPECIFICATION

(11) **EP 1 277 056 B1**
(45) Date of publication and mention of the grant of the patent: **10.10.2007**
(21) Application number: 01924915.0
(22) Date of filing: 11.04.2001
(51) Int. Cl.: G01N 33/545, G01N 33/553, G01N 33/543

(54) **USE OF INK-JET PRINTING TO PRODUCE DIFFRACTION-BASED BIOSENSORS**
VERWENDUNG VON TINTENSTRAHLDRUCK ZUR HERSTELLUNG DER AUF DIFFRAKTION GEGRÜNDETEN BIOSENSOREN
UTILISATION DE L'IMPRESSION PAR JET D'ENCRE AFIN DE PRODUIRE DES BIOCAPTEURS BASES SUR LA DIFFRACTION

(30) Priority: 24.04.2000 US 557453
(43) Date of publication of application: 22.01.2003
(73) Proprietor: Kimberly-Clark Worldwide, Inc., Neenah, WI 54956 (US)
(72) Inventor: KAYLOR, Rosann, M., Cumming, GA 30041 (US); CHIDEBELU-EZE, Chibueze, Obinna, East Point, GA 30344 (US); CHOI, Abraham, B., Duluth, GA 30096 (US)
(74) Representative: Davies, Christopher Robert
(86) International application number: PCT/US2001/011705
(87) International publication number: WO 2001/081921

(56) References cited:
- EP-A- 0 469 445
- WO-A-98/27417
- WO-A-99/31486
- RODA A ET AL: "Protein microdeposition using a conventional ink-jet printer" BIOTECHNIQUES, EATON PUBLISHING, NATICK, US, vol. 28, no. 3, March 2000 (2000-03), pages 492-496, XP002164952 ISSN: 0736-6205
- BLANCHARD A P ET AL: "HIGH-DENSITY OLIGONUCLEOTIDE ARRAYS" BIOSENSORS & BIOELECTRONICS, ELSEVIER SCIENCE PUBLISHERS, BARKING, GB, vol. 11, no. 6/7, 26 April 1996 (1996-04-26), pages 687-690, XP002052193 ISSN: 0956-5663

## Description

### TECHNICAL FIELD

The present invention is generally in the field of detecting analytes in a medium and, more particularly, the present invention relates to sensing devices used to indicate the presence of the analyte in a medium.

### BACKGROUND OF THE INVENTION

There are many systems and devices available for detecting a wide variety of analytes in various media. Most of these systems and devices are relatively expensive and require a trained technician to perform the test. There are many cases where it would be advantageous to be able to rapidly and inexpensively determine if an analyte were present. What is needed is a biosensor system that is easy and inexpensive to manufacture and is capable of reliable and sensitive detection of analytes, including smaller analytes. Additionally, what is needed is an easy, flexible method of preparation of the biosensors which would permit optimum scale-up processing.

Sandstrom et al., 24 Applied Optics 472, 1985, describe use of an optical substrate of silicon with a layer of silicon monoxide and a layer of silicon formed as dielectric films. They indicate that a change in film thickness changes the properties of the optical substrate to produce different colors related to the thickness of the film. The thickness of the film is related to the color observed and a film provided on top of an optical substrate may produce a visible color change. The authors indicate that a mathematical model can be used to quantitate the color change, and that "[c]alculations performed using the computer model show that very little can be gained in optical performance from using a multilayer structure... but a biolayer on the surface changes the reflection of such structures very little since the optical properties are determined mainly by the interfaces inside the multilayer structure. The most sensitive system for detection of biolayers is a single layer coating, while in most other applications performance can be by additional dielectric layers."

Sandstrom et al., go on to indicate that slides formed from metal oxides on metal have certain drawbacks, and that the presence of metal ions can also be harmful in many biochemical applications. They indicate that the ideal top dielectric film is a 2-3 nm thickness of silicon dioxide which is formed spontaneously when silicon monoxide layer is deposited in ambient atmosphere, and that a 70-95 nm layer silicon dioxide on a 40-60 nm layer of silicon monoxide can be used on a glass or plastic substrate. They also describe formation of a wedge of silicon monoxide by selective etching of the silicon monoxide, treatment of the silicon dioxide surface with dichlorodimethylsilane, and application of a biolayer of antigen and antibody. From this wedge construction they were able to determine film thickness with an ellipsometer, and note that the "maximum contrast was found in the region about 65 nm where the interference color changed from purple to blue." They indicate that the sensitivity of such a system is high enough for the detection of protein antigen by immobilized antibodies. They conclude "the designs given are sensitive enough for a wide range of applications." The materials, i.e., glass, silicon, and silicon oxides, are chemically inert and do not affect the biochemical reaction studied. Using the computations above it is possible to design slides that are optimized for different applications. The slides can be manufactured and their quality ensured by industrial methods, and two designs are now commercially available.

U.S. Patent 5,512,131 issued to Kumar et al. describes a device that includes a polymer substrate having a metal coating. An antibody-binding protein layer is stamped on the coated substrate. The device is used in a process for stamping or as a switch. A diffraction pattern is generated when an analyte binds to the device. A visualization device, such as a spectrometer, is then used to determine the presence of the diffraction pattern.

However, the device described by Kumar et al. has several disadvantages. One disadvantage is that an extra visualization device is needed to view any diffraction pattern. By requiring a visualization device, the Kumar et al. device does not allow a large number of samples to be tested since it is not possible to determine the presence of an analyte by using the unaided eye.

U.S. Patent No. 5,482,830 to Bogart, et al., describes a device that includes a substrate which has an optically active surface exhibiting a first color in response to light impinging thereon. This first color is defined as a spectral distribution of the emanating light. The substrate also exhibits a second color which is different from the first color (by having a combination of wavelengths of light which differ from that combination present in the first color, or having a different spectral distribution, or by having an intensity of one or more of those wavelengths different from those present in the first color). The second color is exhibited in response to the same light when the analyte is present on the surface. The change from one color to another can be detected either by use of an instrument, or by eye. Such sensitive detection is an advance over the devices described by Sandstrom and Nygren, supra, and allow use of the devices in commercially viable and competitive manner.

However, the method and device described in the Bogart, et al. patent has several disadvantages. One disadvantage is the high cost of the device. Another problem with the device is the difficulty in controlling the various layers that are placed on the wafer so that one obtains a reliable reading.

The prior art also includes the use of jetting technology for certain applications. For example, in U.S. Patent No. 4,877,745, issued to Hayes et al., a method and apparatus for assaying an analyte is disclosed. The method and apparatus include a jetting chamber used to dispense precise quantities of a diagnostic reagent and a fluid sample onto a substrate. The apparatus uses means to position one of the diagnostic reagent or the fluid sample onto a substrate. Then, the other of the diagnostic reagent or the fluid sample is jetted such that it reacts with the initially positioned fluid. The method uses an electromechanical means to alternatively ink-jet the diagnostic fluid and the sample until a desired quantity of fluids have been dispensed onto a substrate. The method involves positioning a substrate relative to a jetting chamber and, through the use of electrical pulses, applying a diagnostic fluid to the substrate in a predetermined manner. The diagnostic reagents are placed in a collapsible container which changes volume during use of the device. However, since this method prints both the analyte-specific receptor material and the sample to be tested, this method is difficult to operate and requires complex electromechanical means. W09931486 discloses a biosensor comprising a polymer film coated with a metal (e.g. gold), and a patterned binder layer ink-jet printed thereon, comprising e.g. an anti-analyte antibody. The document also discloses methods of producing the biosensor and methods of using the biosensor in detecting analytes.

Accordingly, what is needed is a biosensor device that is easy and inexpensive to manufacture and is capable of reliable and sensitive detection of the analyte to be detected.

### SUMMARY OF THE INVENTION

The present invention provides an inexpensive and sensitive device and method for detecting analytes present in a medium according to the claims. The device includes a biosensing device having a substrate, such as a metalized polymer film, upon which is ink-jet printed a specific predetermined pattern of binder, including antibodies or thiolated antibodies or DNA. Alternatively, antibody-binding proteins such as Protein A or Protein G may be ink-jet printed. Subsequent exposure to the antibody specific for the desired analyte results in patterned deposition of this antibody. Overall, this allows a modular production format such that large rolls of patterned protein may be made for use with different analytes. Then as needed, the final product may be made by exposure to the necessary antibody.

Upon attachment of a target analyte, which is capable of scattering light, to select areas of the polymer film upon which the protein and/or antibody are patterned, diffraction of transmitted and/or reflected light occurs via the physical dimensions and defined, precise placement of the analyte. A diffraction image is produced which can be easily seen with the eye or, optionally, with a sensing device.

The present invention utilizes methods of ink-jet printing of patterned binders. These binders may be antibodies or proteins. If proteins are used, these proteins bind to antibodies to pattern them on the surface as well as maintain the optimum orientation for the receptor antibodies. The receptor antibodies are specific for a particular analyte or class of analyte, depending upon the protein used.

Patterned antibodies cause patterned placement or binding of analytes thereon. The biosensing devices of the present invention produced thereby may be used in one of two ways, depending on the size of the analyte. For analytes which are capable of causing diffraction by themselves, such as microorganisms, the system is used by first exposing the biosensing device to a medium that contains the analyte of choice and then, after an appropriate incubation period, transmitting a light, such as a laser, through the film or reflecting it off of the film. If the analyte is present in the medium and is bound to the patterned antibody layer, the light is diffracted in such a way as to produce a visible image.

Optionally, for very small analytes such as proteins or DNA, the system may utilize "diffraction enhancing elements" which are capable of binding to the target analyte and to the biosensor and are capable of producing a substantial change in the height and/or refractive index, thereby increasing the diffraction efficiency of the biosensor and permitting the detection of smaller analytes. In use, a target analyte attaches either to the diffraction enhancing element, which then attaches to the biosensor, or directly to select areas of the polymer film upon which the antibody is patterned. Then diffraction of transmitted and/or reflected light occurs via the physical dimensions and defined, precise placement of the analyte. A diffraction image is produced which can be easily seen with the eye or, optionally, with a sensing device.

Another option for use of this sensor involves the detection of analytes which are antibodies. The sensing device could include only the patterned antibody-binding proteins, and then would be exposed to the medium plus diffraction enhancing particles that have an antibody specific to the antibody to be detected. The selection of the antibody on the particle is desirably made so that it does not bind non-specifically to the patterned antibody-binding protein, but instead binds only when the analyte antibody is also bound. In this way, the diffraction enhancing elements would cause a substantial change in the height and/or refractive index if the analyte antibody is present, thereby causing a diffraction image to form. It is envisioned that the same format could be used with other immunoassay formats, such as lateral flow assays or microwell plates.

Accordingly, the antibody and antibody layers with the analyte bound thereto can produce optical diffraction patterns to indicate the presence of the analyte. The light can be in the visible spectrum, and be either reflected from the film, or transmitted through it. The light can be a point light source, such as white light or monochromatic electromagnetic radiation in the visible region. The present invention may be used to print directly on a flexible support. The support may include a layer of gold or other suitable metal or metal alloy onto which the antibody is printed.

Still another option for use of this sensor involves the detection of nucleotide-based analytes, such as DNA. This option would utilize the sensing device having patterned oligonucleotides complimentary to the target, which would be exposed to the medium plus diffraction enhancing particles that have oligonucleotides specific to another segment of the target DNA. The nucleotide is then detected in the manner discussed above by looking for a diffraction image.

The present invention provides a low-cost, disposable biosensor which can be mass produced. It includes the use of surfaces patterned with an antibody or an antibody-binding protein. Typically, antibody-binding proteins bind an antibody by its constant region (F_{c}) so that the antibody's antigen-binding regions (F_{ab}) are free for optimum binding activity. The preparation of the patterned protein surfaces also allows maximum flexibility in the sensor production. The final step in production, capturing the desired antibody in the patterned areas, may be done as needed for the desired analyte (i.e. at the time of manufacture).

The biosensors of the present invention can be produced as a single test for detecting an analyte or it can be formatted as a multiple test device. The biosensors of the present invention can be used to detect medical conditions or contamination in garments, such as diapers, and to detect contamination by microorganisms.

As a desired ink-jet printing method, printers having a piezoelectric print-head may be desired when printing temperature-sensitive proteins, such as antibodies.

The present invention can also be used on contact lenses, eyeglasses, window panes, pharmaceutical vials, solvent containers, water bottles, adhesive bandages, and the like to detect contamination.

These and other features and advantages of the present invention will become apparent after a review of the following detailed description of the disclosed embodiments.

### BRIEF DESCRIPTION OF THE FIGURES

Figure 1 shows a top view of a biosensor capable of simultaneously measuring several different analytes in a medium.

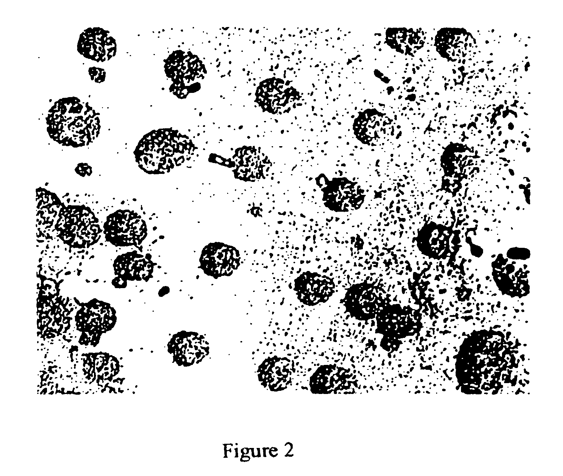

Figure 2 shows a photomicrograph of patterned microparticles in the ink-jet printed features, indicating the presence of an analyte.

### DETAILED DESCRIPTION

The present invention features improved biosensing devices, and methods for preparing and using these biosensing devices, that detect the presence or amount of an analyte of interest within a medium. The analytes that can be detected by the present invention include, but are not limited to, microorganisms such as bacteria, yeasts, fungi and viruses. In contrast to prior devices, those of the present invention allow detection of extremely small quantities of analyte in a medium in a rapid assay lasting only a few minutes. In addition, the biosensing device may be manufactured at much lower cost and at faster speeds than other biosensing devices.

The present invention discloses ink-jet printing of binders onto a polymer film, such as a plastic film. The polymer film may also have a metal coating. "Binders" may include antibodies, including thiolated antibodies, or antibody-binding proteins. The present invention provides an easy method of production suitable for high-speed manufacture. The present invention allows for the development of single use, disposable biosensors based on light diffraction to indicate the presence of the analyte. Upon attachment of a target analyte to select areas of the polymer film which contain the binder, diffraction of transmitted and/or reflected light occurs via the physical dimensions and defined, precise placement of the analyte. For example, yeast, fungi or bacterium are large enough to act as diffraction elements for visible light when placed in organized patterns on a surface. Additionally, the present invention may include diffraction enhancing elements which increase the diffraction efficiency of the biosensor, thereby making it possible to detect any number of different analytes. In addition to producing a simple diffraction image, patterns of analytes can be such as to allow for the development of a holographic sensing image and/or a change in visible color. Thus, the appearance of a hologram or a change in an existing hologram will indicate a positive response. The pattern made by the diffraction of the transmitted light can be any shape including, but not limited to, the transformation of a pattern from one pattern to another upon binding of the analyte to the receptive material. In particularly desired embodiments, the diffraction pattern is discernible in less than one hour after contact of the analyte with the biosensing device of the present invention.

The diffraction grating which produces the diffraction of light upon interaction with the analyte desirably has a refractive index different from that of the surrounding medium. Very small analytes, such as viruses or molecules, can be detected indirectly by using a larger particle that is specific for the small analyte. In one embodiment in which the small analyte can be detected includes coating the particle, such as a latex bead, with a protein material that specifically binds to the analyte of interest. Particles that can be used in the present invention include, but are not limited to, glass, cellulose, synthetic polymers or plastics, latex, polystyrene, polycarbonate, proteins, bacterial or fungal cells and the like. The particles are desirably spherical in shape, but any structural and spatial configuration of the particles may be used in the present invention. For instance, the particles could be slivers, ellipsoids, cubes, and the like. A desirable particle size ranges from a diameter of approximately 0.2 µm to 50.0 µm, desirably between approximately 0.4 µm to 1 µm. The composition of the particle may also vary.

The antibody which is immobilized/patterned on the surface will specifically bind to an epitope on the analyte that is different from the epitope used in the binding to the diffraction enhancing element. Thus, for detecting a medium with a small analyte, such as viral particles, the medium is first exposed to the diffraction enhancing element particles, such as latex particles, to which the viral particles bind. Then, the diffraction enhancing element particles are optionally washed and exposed to the polymer film printed with the virus specific antibodies. The antibodies then bind to the viral particles on the element particle thereby immobilizing the element particles in the same pattern as the antibodies on the film. Because the bound element particles will cause diffraction of the visible light, a diffraction pattern is formed, indicating the presence of the viral particle in the liquid. Additionally, the polymer film may include a metal coating thereon. The printed antibody layer would then be located on the metalized surface of the film.

Alternatively, the analyte may be detected by first exposing the substrate to the medium containing the analyte and causing the analyte to bind to the printed analyte-specific antibody. Next, a solution containing the diffraction enhancing element particles is contacted with the substrate having the analyte bound thereto. The particles then bind to the analyte. Because the bound element particles will cause diffraction of the visible light, a diffraction pattern is formed, indicating the presence of the analyte in the liquid.

In another embodiment, the biosensor, the diffraction enhancing element particles and the medium containing the analyte may be admixed simultaneously. This will result in a combination of the binding procedures discussed above. Some of the analytes will first bond with a diffraction enhancing element particle prior to binding to the substrate. Other analytes will first bind with the substrate and then bind with an element particle. When a point-light source is shone or illuminated through the sensor, a diffraction pattern is formed, indicating the presence of the analyte in the liquid.

Finally, in still another embodiment, the number of steps involved by a user of diffraction diagnostic devices using the diffraction enhancing element particles may be reduced. The approach involves the use of a wicking agent that is used to remove unbound labeled particles, as well as any residual liquid from the sample. The wicking agent then avoids the necessity of any additional rinsing, which may be cumbersome or more difficult for a user. Additionally, a small hole (e.g., 4/32 of an inch) can be punched out of the center of the wicking agent so that once the sample and excess particles are wicked away, the hole allows the user to immediately check for a diffraction image without removing the wicking material. Examples of wicking agents include nitrocellulose membranes, cellulose acetate membranes, and glass microfiber structures.

In addition, the pore size of the membrane may be varied to control the rate and force of wicking. This can affect the accuracy of the diagnostic device, and can also be taken advantage of to create a one-step device. To achieve this, a one-step device having an ink-jet printed capture antibody on a substrate, such as a gold coated polyethylene-terephthalate substrate (gold/MYLAR^{®}), which then has labeled particles pre-dried onto its surface. Additionally, a small pore size membrane (e.g., 0.45 micron nitrocellulose) with a hole cut out is placed on top of the device to complete it. The user simply adds the sample (e.g., serum or blood) to be tested, and then views for a diffraction-image once wicking has occurred. The small pore size delays wicking long enough to allow adequate incubation, such as that needed for antibody-antigen interactions to take place. Alternatively, wicking may be delayed by using an erodible reagent at the periphery of the wicking agent circle. The reagent would eventually dissolve or derivatize so that it would allow wicking after a specific time period.

The analytes that are contemplated as being detected using the present invention include, but are not limited to, bacteria; yeasts; fungi; viruses; rheumatoid factor; antibodies, including, but not limited to IgG, IgM, IgA and IgE antibodies; carcinoembryonic antigen; streptococcus Group A antigen; viral antigens; antigens associated with autoimmune disease; allergens; tumor antigens; streptococcus Group B antigen; HIV I or HIV II antigen; or host response (antibodies) to these and other viruses; antigens specific to RSV or host response (antibodies) to the virus; an antigen; enzyme; hormone; polysaccharide; protein; lipid; carbohydrate; drug or nucleic acid; *Salmonella species; Candida species,* including, but not limited to *Candida albicans* and *Candida tropicalis; Salmonella species; Neisseria meningitides* groups A, B, C, Y and W sub 135, *Streptococcus pneumoniae, E. coli K1, Haemophilus influenza* type *B;* an antigen derived from microorganisms; a hapten, a drug of abuse; a therapeutic drug; an environmental agent; and antigens specific to hepatitis.

In another embodiment of the present invention, nutrients for a specific class of microorganisms can be incorporated into the binder layer. In this way, very low concentrations of microorganisms can be detected by first contacting the biosensor of the present invention with the nutrients incorporated therein and then incubating the biosensor under conditions appropriate for the growth of the bound microorganism. The microorganism is allowed to grow until there are enough organisms to form a diffraction pattern. Of course, in some cases, the microorganism can multiply enough to form a diffraction pattern without the presence of a nutrient on the patterned binder.

A part of the present invention is the method used to pattern receptors, such as antibodies, onto a polymer film or a metalized polymer film. In one part, the method entails ink-jet printing proteins that bind to antibodies. These proteins can include, but are not limited to, Protein A, Protein G, Protein L, as well as their recombinant forms. Commercial versions of these proteins, such as Zymed's (San Francisco, CA) KAPPALOCK^{™}, are also suitable. Thus, the protein material is defined as the base material to create a specific binding pair, with the other part being an antibody specific to the analyte of interest.

In the present invention, a biologically active material, such as the antibody, is printed onto a metalized polymer film, such as a gold/MYLAR^{®} substrate, in a defined pattern, using an ink-jet printer. A resolution of 720 dpi provides an array capable of producing a diffraction image upon binding by a target analyte and labeled microparticles. However, other resolutions may be used as well. The lower resolution of the ink-jet printer still provides adequately small feature sizes (40-100 micron diameter) to give a diffraction image. The antibody may be thiolated, and the resulting phosphate-buffered solution may also have glycerin (up to 40% by weight) added to it in order to prevent spreading of the printed features on the film. The resulting devices may then be used as previously discussed. The presence of the analyte would be indicated if a diffraction image forms. Since diffraction angles are inversely proportional to the feature spacing, it was found that a larger distance from the light source was necessary in order to get a discernible diffraction image. Special viewers may be used to make this more easily detectable.

The receptor material that is bound to the patterned protein is defined by an ability to specifically bind the analyte or analytes of interest. Whatever the selected analyte of interest is, the protein material is designed to bind specifically with the analyte of interest. In the desired embodiments, the biosensing device is configured and arranged to provide a pattern detectable by eye in response to transmission of polychromatic light when the analyte of interest is sandwiched between the antibody and a diffraction enhancing element. In another embodiment, where the analyte is large enough to diffract light, no diffraction enhancing element may be needed.

In many instances, a "blocker" may be necessary to prevent non-specific binding. The term "blocker" as used herein means a reagent that adheres to the sensor surface so that it "blocks" or prevents non-analyte materials from non-specifically binding to the surface (either in the patterned or un-pattemed areas). The blocking step may be done as a post-treatment to a surface which has already been ink-jet printed ("post-block"), and is the standard technique for filling in non-ink-jet printed regions with another thiol. However, the inventors have discovered that a "pre-block" technique is desired over the post-block technique. In the pre-block technique, the surface of the substrate is pre-treated with a non-thiol containing blocker and then ink-jet printed. Not wishing to be bound to any theory, it is theorized that the ink-jet printed material (usually sulfur containing) displaces the physisorbed blocker, thereby permitting the printed material to be bound directly to the surface of the substrate. A subsequent post-block may also be performed, if desired. Blockers can include, but are not limited to, β-casein, albumins such as bovine serum albumin, pluronic or other surfactants, polyethylene glycol, polyvinyl pyrrolidone, polyvinyl alcohol, or sulfur derivatives of the above compounds, and any other blocking material known to those of ordinary skill in the art.

The matrix containing the analyte of interest may be a solid, a gas, or a bodily fluid such as an interstitial fluid, mucous, saliva, urine, fecal material, tissue, marrow, cerebral spinal fluid, serum, plasma, whole blood, synovial sputum, buffered solutions, extracted solutions, semen, vaginal secretions, pericardial, gastric, peritoneal, pleural, a throat swab or other washes and the like. The analyte of interest may be an antigen, an antibody, an enzyme, a toxin, an environmental agent, a cytoplasm component, pili or flagella component, protein, polysaccharide, drug, or any other material that is capable of being recognized by an antibody. For example, receptor material for bacteria may specifically bind a surface membrane component, protein or lipid, a polysaccharide, a nucleic acid, or an enzyme. The analyte which is indicative of the bacteria may be a saccharide or polysaccharide, an enzyme, a nucleic acid, a membrane component, a ganglioside or an antibody produced by the host in response to the bacteria. The presence of the analyte may indicate an infectious disease (bacterial or viral), cancer, an allergy, or other medical disorder or condition. The presence of the analyte may be an indication of water or food contamination or other harmful materials. The analyte may indicate drug abuse or may monitor levels of therapeutic agents.

In some cases, the analyte may not simply bind the receptor material, but may cause a detectable modification of the receptor material to occur. This interaction could cause an increase in mass at the test surface or a decrease in the amount of receptor material on the test surface. An example of the latter is the interaction of a degradative enzyme or material with a specific, immobilized substrate. In this case, one would see a diffraction pattern before interaction with the analyte of interest, but the diffraction pattern would disappear if the analyte were present. The specific mechanism through which binding, hybridization, or interaction of the analyte with the receptor material occurs is not important to this invention, but may impact the reaction conditions used in the final assay protocol.

The medium in which the analyte may reside can be solid, gel-like, liquid or gas. For purposes of detecting an analyte in a body fluid, the fluid is selected from, but not limited to, urine, serum, plasma, spinal fluid, sputum, whole blood, saliva, urogenital secretions, fecal extracts, pericardial, gastric, peritoneal, pleural washes, vaginal secretions, or a throat swab; and the method optionally includes using a spectrophotometer to measure the appearance of the refractive pattern. The most common gas that is contemplated as being used with the biosensing device of the present invention is air.

The biosensing device of the present invention utilizes methods of ink-jet printing of patterned binders on substrates, desirably metalized polymer films, the subsequent exposure to an antibody to achieve a patterned antibody, the compositions produced thereby, and the use of these compositions. Patterned binder layers allow for the controlled placement of antibodies thereon which can bind an analyte. The term "patterned binder layers" as used herein means the binder, such as a protein or antibody, plus the desired antibody layers in any pattern on the substrates including a solid pattern.

When the film with the patterned binder layers is exposed to an analyte that is capable of reacting with the antibody, the film will produce optical diffraction patterns which differ depending on the reaction of the antibody with the analyte of interest. The liquid may be a high surface tension fluid such as water. The light can be in the visible spectrum, and be either reflected from the film, or transmitted through it, and the analyte can be any compound reacting with the patterned binder layer.

In desired embodiments, the method involves contacting the substrate with a test sample potentially containing the analyte under conditions in which the substrate causes a change in the refractive index. When light is transmitted through the film with the patterned binder layer, a visible diffraction pattern is formed and can be visualized by directing the light to a surface or by looking directly through the substrate.

Alternatively, the diffraction image can be visualized with the aid of a simple viewing device consisting of a point light source and optical components such as lenses and mirrors. This viewer could be designed specifically for the samples obtained from ink-jet printing methods. If designed properly, the viewer is able to provide an image from a hand-held device (e.g., ~6-inch dimension) that is comparable to an image obtained from a much longer view path length without the viewer (e.g., ~5 feet). A longer viewing path length will usually be needed without the viewer due to the larger spacing obtained from an ink-jet printer (>50 micron period) as compared to prior art contact printing methods.

In one embodiment, the present invention is contemplated in a dipstick form in which the ink-jet printed film is mounted at the end of the dipstick. In use the dipstick is dipped into the liquid in which the suspected analyte may be present and allowed to remain for several minutes. The dipstick is then removed and then, either a light is projected through the film or the film is observed with a light behind the film. If a pattern is observed, then the analyte is present in the liquid.

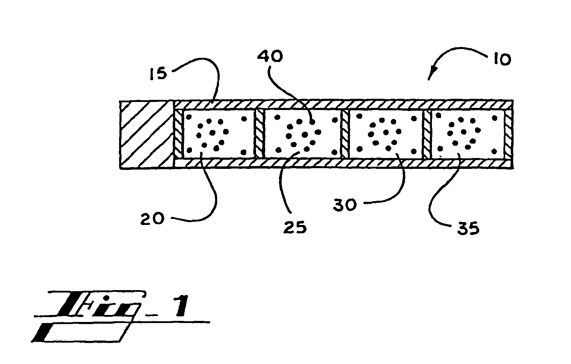

In another embodiment of the present invention, a single support strip may be used to test for multiple analytes. As shown in Figure 1, a strip **10** is provided with several ink-jet printed films **20, 25, 30** and **35**, each film having a pattern **40** printed thereon. Each of the ink-jet printed films **20, 25, 30**, and **35** may be a metalized polymer film with each film having a different antibody thereon that is specific for a different analyte. It can be seen that the present invention can be formatted in any array with a variety of ink-jet printed films thereby allowing the user of the biosensor device of the present invention to detect the presence of multiple analytes in a medium using a single support.

In yet another embodiment of the present invention, the biosensor can be attached to an adhesively backed sticker or decal which can then be placed on a hard surface or container wall. The biosensor can be placed on the inside surface of a container such as a food package or a glass vial. The biosensor can then be visualized to determine the presence of analyte.

Typically, a gold film, 5 to 2000 nm thick, is supported on a polyethylene-terephthalate film, Si/SiO₂ wafer or glass sheet. An adhesion promoter, such as titanium, may also be used as an adhesion promoter between gold and the support. The binder attaches to the gold surface during ink-jet printing.

A more detailed description of the methods and compositions of the present invention follows. All publications cited herein are incorporated by reference in their entirety.

Any polymer film is suitable for the present invention. Desirably, the polymer film is a plastic film. More desirably, the polymer film is also capable of having a metal coating deposited thereon. These include, but are not limited to polymers such as: polyethylene-terephthalate (MYLAR^{®}), acrylonitrile-butadiene-styrene, acrylonitrile-methyl acrylate copolymer, cellophane, cellulosic polymers such as ethyl cellulose, cellulose acetate, cellulose acetate butyrate, cellulose propionate, cellulose triacetate, cellulose triacetate, polyethylene, polyethylene - vinyl acetate copolymers, ionomers (ethylene polymers) polyethylene-nylon copolymers, polypropylene, methyl pentene polymers, polyvinyl fluoride, and aromatic polysulfones. Desirably, the plastic film has an optical transparency of greater than 80%. Other suitable thermoplastics and suppliers may be found, for example, in reference works such as the Modern Plastics Encyclopedia (McGraw-Hill Publishing Co., New York 1923-1996).

In one embodiment of the invention, the polymer film has a metal coating thereon and the resulting metalized polymer film has an optical transparency of between approximately 5% and 95%. A more desired optical transparency for the polymer film used in the present invention is between approximately 20% and 80%. In a desired embodiment of the present invention, the polymer film has at least an approximately 80% optical transparency, and the thickness of the metal coating is such as to maintain an optical transparency greater than about 20%, so that diffraction patterns can be produced by either reflected or transmitted light. The optical transparency is based upon the visible transmittance of wavelengths of visible light passing through the film. This level of optical transparency corresponds to a metal coating thickness of about 10 nm. However, in other embodiments of the invention, the gold thickness may be between approximately 1 nm and 1000 nm.

The desired metal for deposition on the film is gold. However, other metals may be used including, but not limited to, silver, aluminum, chromium, copper, iron, zirconium, platinum and nickel, as well as oxides of these metals.

In another embodiment, the invention features an optical assay device, having an optically active receptive surface configured and arranged to allow simultaneous assay of a plurality of samples on the surface for one analyte of interest, and an automated liquid handling apparatus (e.g., a pipetting device) configured and arranged to dispense sample and reagent solutions to the surface.

Below is provided a description by which the optimal materials and methods useful for construction of optical test surfaces of this invention can be made. Generally, the present invention includes novel optically active test surfaces for the direct detection of an analyte. These test surfaces have an analyte-specific antibody printed in a specific pattern to the test surface. The detection method involves contacting this device with a sample fluid containing the analyte of interest, and then examining the change in diffraction of transmitted or reflected light by observing whether a diffraction pattern is formed.

Alternatively, the analyte-specific antibody is bound by use of an attachment layer - namely the antibody-binding proteins. Thus, the invention would then provide a detection device which includes selecting an optical substrate, printing a pattern of antibody-binding protein, and then exposing this to the antibody for the desired analyte.

In one instance, the analyte may be a nucleotide-based analyte, such as DNA. A complementary oligonucleotide to the target DNA is ink-jet printed on the substrate and then the sensor may optionally blocked with a blocking material, such as β-casein, an albumin, a surfactant, polyethylene glycol, polyvinyl alcohol, or sulfur derivatives thereof, rinsed and air-dried. The sensor is then placed in an analyte solution containing a DNA strand of interest. Next, diffraction enhancing element particles with oligonucleotide complimentary to another area of the analyte DNA are added to the sensor. The sensor and particles may then be heated and then rinsed. After this, a point light source may be transmitted or reflected through the sensor sample. A diffraction image would be seen on the other side of the light beam in the presence of the DNA analyte.

The present invention has a broad range of applications and may be utilized in a variety of specific binding pair assay methods. For example, the devices of this invention can be used in immunoassay methods for either antigen or antibody detection. The devices may be adapted for use in direct, indirect, or competitive detection schemes.

In one embodiment of the present invention, the antibody-binding protein layer has the following general formula:

X-P-Ab

X is optional as a means of allowing chemisorption to a metal or metal oxide. For example, X may be asymmetrical or symmetrical disulfide (-R'SSY', -RSSY), sulfide (-R'SY', -RSY), diselenide (-R'Se-SeY'), selenide (-R'SeY', -RSeY), thiol (-SH), nitrile (-CN), isonitrile, nitro (-NO₂), selenol (-SeH), trivalent phosphorous compounds, isothiocyanate, xanthate, thiocarbamate, phosphine, thioacid or dithioacid, carboxylic acids, hydroxylic acids, and hydroxamic acids.

P represents the antibody-binding proteins which may be derivatized with X. Ab represents the antibody specific to the desired analyte.

The sensing device includes a substrate onto which the binder material, such as an antibody or protein, is applied to the entire surface of the substrate (e.g. by dipping or spraying the entire surface). The binder covers the entire surface of the substrate, such as a polymer film or metal-coated polymer film. Next, an inactivating agent, such as protease or a strongly anionic surfactant, such as sodium dodecyl sulfate (SDS), is printed onto the binder-coated substrate in a manner that results in a portion of the binder material being inactivated. The inactivated portion is formed in a preselected pattern on the substrate. The analyte binds only to the active portion of the binder material. Transmitted or reflected light results in the formation of a diffraction pattern that may be seen by the unaided eye or, desirably, with a specially designed viewing device.

### EXAMPLES

### EXAMPLE 1

This example is directed to the thiolation procedure for proteins (e.g., antibodies or antibody-binding proteins such as protein A).

To 1 mg of protein was added 450 µL phosphate buffered saline (PBS, made using Pierce Catalog #28372 as per their instructions to give pH 7.2 with 0.1 M sodium phosphate and 0.15 M sodium chloride). Next, 50 µL of a 10mM aqueous solution of Sulfo-LC-SPDP (Pierce Catalog # 21650) was added. This mixture is allowed to react for 60 minutes at room temperature, then poured into a D-Salt Polyacrylamide desalting column (Pierce, 5 mL bed volume equilibrated with appropriate buffer). An acetate buffer, made with 0.1 M sodium acetate and 0.1 M sodium chloride adjusted to pH 4.5, was used if a subsequent reduction of the disulfide bond was done, while a PBS buffer, pH 7.2, was used if the protein derivative was to be left in the disulfide form. [Note: Both forms have been found to be suitable in making biosensing devices, although the desirred form is that prepared from the PBS buffer since it does not require the subsequent reduction step.] Fractions of 500 µL each were collected from the column, and the fraction with protein derivative was determined using a COOMASSIE^{®} Plus Protein Assay.

### EXAMPLES 2-7

Examples 2 to 7 are directed to making a sensing device using ink-jet printing techniques.

### EXAMPLE 2

Gold-coated polyethylene-terephthalate (MYLAR^{®}) was printed at 720 dpi with a thiolated polyclonal antibody to Strep B (e.g., Cat # M-C664-50L from Lee Laboratories; Grayson, GA). The gold-coated MYLAR^{®} was a "Icl 453 / Au-20" / coating, which was a 200 gauge polyethylene-terephthalate film with a gold coating that provided an average surface resistance of 19-20 ohms per square (+ 20%) from Courtaulds Performance Films (Canoga Park, California). The printer was an ink jet printer (e.g., Epson Stylus 640 or 740 printers) with settings of: Paper = Photo paper; Ink = Black ink only; Print Quality = 720 dpi; Halftoning = No Halftoning. A 20 MB file of an x,y array set to 1500 pixels per inch resolution (Adobe Photoshop) is one example. Upon printing, this produced a patterned x,y array of antibody in features ranging from 40-100 microns in diameter, which is adequate to diffract upon analyte deposition. The resulting printed sample was exposed to 34 microliters of Strep B antigen-spiked buffer solution (e.g., phosphate buffered saline at pH 7.2; antigen = Difco Cat# 2979-50) by placing the 34 microliters as a droplet on top of the printed film. After 5 minutes, 11 microliters of 0.5 micron particles conjugated with polyclonal antibody to Strep B were added by pipetting the 11 microliters directly on top of the droplet containing the antigen; the particles were suspended to ~10% solids in a saline / Tween 20 solution containing beta casein (e.g., 0.005-0.01 % Tween 20, 0.001-0.01 M NaCl, and 5 mg/mL beta casein). After 10 minutes incubation, a disk of nitrocellulose (e.g., 0.8 micron pore size) having a hole in the center (e.g., 4/32-inch diameter) is placed on top of the liquid/particle mixture. This wicks away unbound particles and excess liquid, so that the sample can be checked for a diffraction image using a point light source, such as a laser. The light is aimed to shine through the hole such that a diffraction image is seen if the analyte is present. Alternately, a small, hand-held viewing device that uses mirrors and a microscope objective lens could also be used to display a diffraction image if the analyte was present.

### EXAMPLE 3

The gold-coated polyethylene-terephthalate film of Example 2 was printed at 720 dpi with a thiolated monoclonal antibody to IgE (e.g., such as one specific to the C3-C4 domains of IgE). The printer was an ink jet printer (e.g., Epson Stylus 640 or 740 printers) with settings of: Paper = Photo paper; Ink = Black ink only; Print Quality = 720 dpi; Halftoning = No Halftoning. A 20 MB file of an x,y array set to 1500 pixels per inch resolution (Adobe Photoshop) is one example. Upon printing, this produced a patterned x,y array of antibody in features ranging from 40-100 microns in diameter, which is adequate to diffract upon analyte deposition.

### EXAMPLE 4

The gold-coated polyethylene-terephthalate film of Example 2 was printed at 1440 dpi with a thiolated monoclonal antibody to IgE (e.g., such as one specific to the C3-C4 domains of IgE). The printer was an ink jet printer (e.g., Epson Stylus 640 or 740 printers) with settings of: Paper = Photo paper; Ink = Black ink only; Print Quality = 720 dpi; Halftoning = No Halftoning. A 20 MB file of an x,y array set to 1500 pixels per inch resolution (Adobe Photoshop) is one example. Upon printing, this produced a patterned x,y array of antibody in features ranging from 40-100 microns in diameter, which is adequate to diffract upon analyte deposition.

### EXAMPLE 5

The gold-coated polyethylene-terephthalate film of Example 2 was printed at 720 dpi with a thiolated monoclonal antibody to IgE (e.g., such as one specific to the C3-C4 domains of IgE). The printer was an ink jet printer (e.g., Epson Stylus 640 or 740 printers) with settings of: Paper = Photo paper; Ink = Black ink only; Print Quality = 720 dpi; Halftoning = No Halftoning. A 20 MB file of an x,y array set to 1500 pixels per inch resolution (Adobe Photoshop) is one example. Upon printing, this produced a patterned x,y array of antibody in features ranging from 40-100 microns in diameter, which is adequate to diffract upon analyte deposition. The resulting printed sample was exposed to 34 microliters of IgE-spiked buffer solution (e.g., phosphate buffered saline at pH 7.2; IgE = human polyclonal IgE from Biodesign, Cat#A10164H) by placing the 34 microliters as a droplet on top of the printed film. After 5 minutes, 11 microliters of 0.5 micron particles conjugated with *Aspergillus fumigatus* allergen extract (e.g., from Greer Laboratories) were added by pipetting the 11 microliters directly on top of the droplet containing the antigen; the particles were suspended to ~10% solids in a saline / Tween 20 solution containing beta casein (e.g., 0.005-0.01% Tween 20, 0.001-0.01M NaCl, and 5 mg/mL beta casein). After 10 minutes incubation, a disk of nitrocellulose (e.g., 0.8 micron pore size) having a hole in the center (e.g., 3/32-inch diameter) is placed on top of the liquid/particle mixture. This wicks away unbound particles and excess liquid, so that the sample can be checked for a diffraction image using a point light source, such as a laser. The light is aimed to shine through the hole such that a diffraction image is seen if the analyte is present. Alternately, a small, hand-held viewing device that uses mirrors and a microscope objective lens could also be used to display a diffraction image if the analyte was present.

### EXAMPLE 6

The gold-coated polyethylene-terephthalate film of Example 2 was printed at 720 dpi with a thiolated monoclonal antibody to IgE (e.g., such as one specific to the C3-C4 domains of IgE). The printer was an ink jet printer (e.g., Epson Stylus 640 or 740 printers) with settings of: Paper = Photo paper; Ink = Black ink only; Print Quality = 720 dpi; Halftoning = No Halftoning. A 20 MB file of an x,y array set to 1500 pixels per inch resolution (Adobe Photoshop) is one example. Upon printing, this produced a patterned x,y array of antibody in features ranging from 40-100 microns in diameter, which is adequate to diffract upon analyte deposition. The resulting printed sample was exposed to 34 microliters of IgE-spiked buffer solution (e.g., phosphate buffered saline at pH 7.2; IgE = human polyclonal IgE from Biodesign, Cat#A10164H) by placing the 34 microliters as a droplet on top of the printed film. After 5 minutes, 11 microliters of 0.5 micron particles conjugated with *Aspergillus fumigatus* allergen extract (e.g., from Greer Laboratories) were added by pipetting the 11 microliters directly on top of the droplet containing the antigen; the particles were suspended to ~10% solids in a saline / Tween 20 solution containing beta casein (e.g., 0.005-0.001% Tween 20, 0.001-0.01M NaCl, and 5 mg/mL beta casein). After 10 minutes incubation, a disk of nitrocellulose (e.g., 0.8 micron pore size) having a hole in the center (e.g., 3/32-inch diameter) is placed on top of the liquid/particle mixture. This wicks away unbound particles and excess liquid, so that the sample can be checked for a diffraction image using a point light source, such as a laser. The light is aimed to shine through the hole such that a diffraction image is seen if the analyte is present. Alternately, a small, hand-held viewing device that uses mirrors and a microscope objective lens could also be used to display a diffraction image if the analyte was present.

### EXAMPLE 7

The gold-coated polyethylene-terephthalate film of Example 2 was pre-treated with a 1% polyvinylpyrrolidone (e.g., Cat# PVP-10 from Sigma, St. Louis, MO) distilled water solution for 10 minutes, and then rinsed with distilled water. Alternatively, the PVP solution could also contain 1% Triton (e.g., Triton X-100) surfactant. The pre-treated film was then loaded in the paper tray of an ink jet printer (e.g., Epson Stylus 640 or 740). A solution of thiolated monoclonal antibody to IgE (such as one specific to the C3-C4 domains of IgE, e.g., Cat #Z86410 from Biodesign; Kennebunk, Maine) containing 10-30% glycerin was placed in an empty "black ink" cartridge, and printed on the gold/MYLAR at 720 dpi using the following parameters:
Paper --Photo paper
Ink -- Black only
Print Quality --Fine 720dpi
Halftoning -- no halftoning

A pattern, such as one created through Adobe Photoshop 3.0 of an x,y array of pixels (resolution of 1500 pixels per inch, total size 20 megabytes), was printed on the gold/MYLAR. It was found that the glycerine helped provide smaller printed circles (e.g., typically 40-100 um in size), by preventing the droplet from spreading as it was printed on the PVP-treated gold surface. Upon printing, this produced a patterned x,y array of antibody in features ranging from 40-100 microns in diameter, which is adequate to diffract upon analyte deposition. The resulting printed sample was exposed to 34 microliters of IgE-spiked buffer solution (e.g., phosphate buffered saline at pH 7.2; IgE = human polyclonal IgE from Biodesign, Cat# A10164H) by placing the 34 microliters as a droplet on top of the printed film. After 5 minutes, 11 microliters of 0.5 micron particles conjugated with *Aspergillus fumigatus* allergen extract (e.g., from Greer Laboratories) were added by pipetting the 11 microliters directly on top of the droplet containing the antigen; the particles were suspended to ~10% solids in a saline / Tween 20 solution containing beta casein (e.g., 0.005-0.01% Tween 20, 0.001-0.01 M NaCl, and 5 mg/mL beta casein). After 20 minutes incubation, a disk of nitrocellulose (e.g., 0.8 micron pore size) having a hole in the center (e.g., 3/32-inch diameter) is placed on top of the liquid/particle mixture. This wicks away unbound particles and excess liquid, so that the sample can be checked for a diffraction image using a point light source, such as a laser. The light is aimed to shine through the hole such that a diffraction image is seen if the analyte is present. Alternately, a small, hand-held viewing device that uses mirrors and a microscope objective lens could also be used to display a diffraction image if the analyte was present.

## Claims

1. A biosensor comprising:
a polymer film comprising a metal coating; and
a binder layer created entirely over the metal-coated polymer film and then ink-jet printed such that a patterned portion of the binder layer is inactivated.

2. The biosensor of claim 1, further wherein the binder layer has an antibody thereon that is specific for an analyte.

3. The biosensor of claim 1 or claim 2, wherein the binder layer has been inactivated in a pattern by ink-jet printing a substance capable of destroying its analyte-binding activity, wherein the substance is selected from a protease or a strongly anionic surfactant.

4. A method of making a biosensor comprising:
coating a layer of binder material on a metal-coated polymer film; and
ink-jetting an inactivating material to inactivate a patterned portion of the binder layer.

5. The method of claim 4, wherein the binder material layer has an antibody thereon that is specific for an analyte.

6. The method of claim 4 or claim 5, wherein the binder layer has been inactivated in a pattern by ink-jet printing a substance capable of destroying its analyte-binding activity, wherein the substance is selected from a protease or sodium dodecyl sulfate.

7. A method of detecting an analyte in a medium comprising:
contacting the medium suspected of containing the analyte with a biosensing device, the biosensing device comprising:
a polymer film comprising a metal coating; and
a binder layer created entirely over the metal-coated polymer film and then ink-jet printed such that a patterned portion of the binder layer is inactivated; and
transmitting a light through the polymer film or reflecting a light from the polymer film; and
detecting presence of the analyte bound to the binder layer by detecting a pattern formed by diffraction of the transmitted light or the reflected light.

8. The method of claim 7, wherein the binder material layer has an antibody thereon that is specific for an analyte.

9. The method of claim 7 or claim 8, wherein the binder layer has been inactivated in a pattern by ink-jet printing a substance capable of destroying its analyte-binding activity, wherein the substance is selected from a protease or sodium dodecyl sulfate.

## Patentansprüche

1. Biosensor umfassend:
einen Polymerfilm, der einen Metallüberzug umfasst; und
eine Bindemittelschicht, die über dem gesamten metallbeschichteten Polymerfilm erzeugt wurde und dann mittels Tintenstrahlaufzeichnung bedruckt wurde, so dass ein gemusterter Teil der Bindemittelschicht inaktiviert ist.

2. Biosensor nach Anspruch 1, worin ferner ein Antikörper, der für einen Analyten spezifisch ist, auf die Bindemittelschicht aufgebracht ist.

3. Biosensor nach Anspruch 1 oder Anspruch 2, worin die Bindemittelschicht durch Aufdrucken einer Substanz, die ihre Analytenbindungs-Aktivität zerstören kann, mittels Tintenstrahlaufzeichnung nach einem Muster inaktiviert wurde und worin die Substanz ausgewählt ist aus einer Protease oder einem stark anionischen, oberflächenaktiven Stoff.

4. Verfahren zum Herstellen eines Biosensors, umfassend:
Beschichten eines metallbeschichteten Polymerfilms mit einer Schicht aus einem Bindemittelmaterial; und
Aufdrucken eines inaktivierenden Materials mittels Tintenstrahlaufzeichnung, um einen gemusterten Teil der Bindemittelschicht zu inaktivieren.

5. Verfahren nach Anspruch 4, worin ein Antikörper, der für einen Analyten spezifisch ist, auf die Schicht aus dem Bindemittelmaterial aufgebracht ist.

6. Verfahren nach Anspruch 4 oder Anspruch 5, worin die Bindemittelschicht durch Aufdrucken einer Substanz, die ihre Analytenbindungs-Aktivität zerstören kann, mittels Tintenstrahlaufzeichnung nach einem Muster inaktiviert wurde und worin die Substanz ausgewählt ist aus einer Protease oder Natriumdodecylsulfat.

7. Verfahren zum Nachweisen eines Analyten in einem Medium, umfassend:
das In-Kontakt-Bringen des Mediums, das vermutlich den Analyten enthält, mit einer Biosensorvorrichtung, wobei die Biosensorvorrichtung umfasst:
einen Polymerfilm, der einen Metallüberzug umfasst; und
eine Bindemittelschicht, die über dem gesamten, metallbeschichteten Polymerfilm erzeugt wurde und dann mittels Tintenstrahlaufzeichnung bedruckt wurde, so
dass ein gemusterter Teil der Bindemittelschicht inaktiviert ist; und
Durchsenden eines Lichts durch den Polymerfilm oder Reflektieren eines Lichts von dem Polymerfilm; und
Nachweisen des Vorhandenseins des an die Bindemittelschicht gebundenen Analyten durch Nachweisen eines durch Beugung des durchgesendeten Lichts oder des reflektierten Lichts ausgebildeten Musters.

8. Verfahren nach Anspruch 7, worin ein Antikörper, der für einen Analyten spezifisch ist, auf die Schicht aus dem Bindemittelmaterial aufgebracht ist.

9. Verfahren nach Anspruch 7 oder Anspruch 8, worin die Bindemittelschicht durch Aufdrucken einer Substanz, die ihre Analytenbindungs-Aktivität zerstören kann, mittels Tintenstrahlaufzeichnung nach einem Muster inaktiviert wurde und worin die Substanz ausgewählt ist aus einer Protease oder Natriumdodecylsulfat.

## Revendications

1. Biocapteur comprenant :
un film polymère comprenant un revêtement métallique ; et
une couche de liant créée entièrement sur le film polymère revêtu de métal puis imprimée par jet d'encre de telle sorte qu'une portion de la couche de liant est inactivée selon un motif.

2. Biocapteur selon la revendication 1, dans lequel, en outre, la couche de liant comporte, en surface, un anticorps qui est spécifique à un analyte.

3. Biocapteur selon la revendication 1 ou la revendication 2, dans lequel la couche de liant a été inactivée selon un motif en imprimant, par jet d'encre, une substance capable de détruire son activité de liaison de l'analyte, la substance étant sélectionnée entre une protéase et un tensio-actif fortement anionique.

4. Procédé de fabrication d'un biocapteur, comprenant :
le revêtement par une couche de matériau liant d'un film polymère revêtu de métal ; et
l'impression par jet d'encre d'un matériau d'inactivation pour inactiver, selon un motif, une portion de la couche de liant.

5. Procédé selon la revendication 4, dans lequel la couche de matériau liant comporte, en surface, un anticorps spécifique à un analyte.

6. Procédé selon la revendication 4 ou la revendication 5, dans lequel la couche de liant a été inactivée selon un motif par impression, par jet d'encre, d'une substance capable de détruire son activité de liaison de l'analyte, la substance étant sélectionnée entre une protéase et le docécylsulfate de sodium.

7. Procédé de détection d'un analyte dans un milieu, comprenant :
la mise en contact du milieu suspecté de contenir l'analyte avec un dispositif biocapteur, le dispositif biocapteur comprenant :
un film polymère comprenant un revêtement métallique; et
une couche de liant créée entièrement sur le film polymère revêtu de métal puis imprimée par jet d'encre de telle sorte qu'une portion de la couche de liant est inactivée selon un motif.
la transmission d'une lumière au travers du film polymère ou la réflexion d'une lumière depuis le film polymère ; et
la détection de la présence de l'analyte lié à la couche de liant par détection d'un motif formé par diffraction de la lumière transmise ou de la lumière réfléchie.

8. Procédé selon la revendication 7, dans lequel la couche de matériau liant comporte, en surface, un anticorps qui est spécifique à un analyte.

9. Procédé selon la revendication 7 ou la revendication 8, dans lequel la couche de liant a été inactivée selon un motif par impression, par jet d'encre, d'une substance capable de détruire son activité de liaison de l'analyte, la substance étant sélectionnée entre une protéase et le dodécylsulfate de sodium.
